# EUROPÄISCHE PATENTANMELDUNG

(11) **EP 3 761 105 A2**
(43) Veröffentlichungstag der Anmeldung: **06.01.2021**
(21) Anmeldenummer: 20196068.9
(22) Anmeldetag: 14.09.2020
(51) Int. Cl.: G02C 7/08, G02C 9/04, A61B 90/50, F16B 2/06, F16B 2/12, F16B 5/06, G02C 11/04

(54) **KLEMMMECHANISMUS ZUM BEFESTIGEN EINES SCHUTZSCHILDES AN EINE BRILLE, SCHUTZ-SCHILDVORRICHTUNG UND BRILLE MIT EINER SOLCHEN SCHUTZSCHILDVORRICHTUNG**

(30) Priorität: 30.03.2020 DE 202020101699 U
(71) Anmelder: MB-Technics GmbH, 85567 Grafing (DE)
(72) Erfinder: Die Erfindernennung liegt noch nicht vor
(74) Vertreter: HGF

(57) **Zusammenfassung**

Die Erfindung betrifft einen Klemmmechanismus zum Befestigen eines Schutzschildes (2) an einer Brille (3), umfassend
eine Klemme (12, 13) zum Klemmen an einen Brillenbügel (5), wobei die Klemme einen inneren und einen äußeren Klemmbacken (12, 13) aufweist und der äußere Klemmbacken (13) zur Anordnung an der Außenseite eine Brillenbügels (5) vorgesehen ist,
eine stiftförmige Halteeinrichtung (25, 28) zum Durchgreifen einer Durchgangsbohrung in einem Schutzschild (2), und
ein Fixierelement (26, 29) zum Fixieren des Schutzschildes (2) am Klemmmechanismus (3). Nach einem ersten Aspekt zeichnet sich der Klemmmechanismus dadurch aus, dass die beiden Klemmbacken (12, 13) derart zusammensteckbar sind, dass sie in Längsrichtung der stiftförmigen Halteeinrichtung (25, 28) zueinander verschieblich gelagert sind. Nach einem zweiten Aspekt ist einer der beiden Klemmbacken (12, 13) und insbesondere der äußere Klemmbacken (13) mit zwei vertikalen Klemmbackenabschnitten (20, 21) ausgebildet, welche jeweils eine Klemmfläche (24) bilden, so dass der andere Klemmbacken (13, 12) mit einer Klemmfläche (17) im Bereich zwischen den beiden Klemmflächen (24) der Klemmbackenabschnitte (20, 21) angeordnet ist.

## Beschreibung

Die vorliegende Erfindung betrifft einen Klemmmechanismus zum Befestigen eines Schutzschildes an eine Brille, insbesondere zum Befestigen an eine Lupenbrille, eine Schutzschildvorrichtung zur Befestigung eines Schutzschildes an einer Brille, insbesondere an einer Lupenbrille sowie eine Lupenbrille, mit einer Schutzschildvorrichtung.

Ärzte, insbesondere Chirurgen und Zahnärzte, verwenden oft Lupenbrillen bei der Diagnose oder Operation, um möglichst präzise den jeweiligen Patienten untersuchen zu können. Eine Lupenbrille ist eine Brille, bei welcher an den Brillengläsern jeweils eine Lupe oder ein Fernrohr befestigt ist, so dass der Träger der Brille durch die Lupe bzw. das Fernrohr hindurchsehen kann. Optional können die Lupenbrillen auch mit einer Beleuchtungseinrichtung versehen sein, so dass die mit den Lupen bzw. Ferngläsern betrachtbaren Bereiche optimal ausgeleuchet sind.

Um sich bei Operationen oder bei der Diagnose von Patienten vor ansteckenden Viren oder Keimen zu schützen, tragen die Ärzte oft einen Mundschutz, der sich auch über die Nase erstrecken sollte.

Es besteht ein erheblicher Bedarf beim Tragen von Lupenbrillen den Infektionsschutz zu verbessern und sich vor Flüssigkeitsspritzer und insbesondere Blutspritzer zu schützen und die Aerosolbelastung im Gesichtsbereich zu reduzieren.

Aus der US 4,965,887 geht ein an einer Brille befestigbares Schutzschild hervor, das zwei elastische Klemmen aufweist. Mit den Klemmen kann das Schutzschild an Brillenbügel der Brille befestigt werden. Die elastische Klemme ist mit einer Feder beaufschlagt oder kann mit einer Schraube zum Fixieren versehen sein.

Der Erfindung liegt die Aufgabe zugrunde, Mittel bereitzustellen, mit welchen Ärzte sich einfach und angenehm vor Flüssigkeitsspritzer und Blutspritzer bei einer Operation oder bei einer Diagnose an einem Patienten schützen können und die Benutzung einer Lupenbrille in keinster Weise beeinträchtigt wird.

Die Aufgabe wird durch die Gegenstände der unabhängigen Ansprüche gelöst. Vorteilhafte Ausgestaltungen sind in den jeweiligen Unteransprüchen angegeben.

Erfindungsgemäß wird ein Klemmmechanismus zum Befestigen eines Schutzschildes an einer Brille, insbesondere an einer Lupenbrille, vorgesehen, umfassend
- eine Klemme zum Klemmen an einen Brillenbügel, wobei die Klemme einen inneren und einen äußeren Klemmbacken aufweist und der äußere Klemmbacken (13) zur Anordnung an der Außenseite eine Brillenbügels
   vorgesehen ist,
- eine stiftförmige Halteeinrichtung zum Durchgreifen einer Durchgangsbohrung in einem Schutzschild, und
- ein Fixierelement zum Fixieren des Schutzschildes am Klemmmechanismus.

Nach einem ersten Aspekt zeichnet sich der Klemmmechanismus dadurch aus, dass einer der beiden Klemmbacken und insbesondere der äußere Klemmbacken mit zwei vertikalen Klemmbackenabschnitten ausgebildet ist, welche jeweils eine Klemmfläche bilden, so dass der andere Klemmbacken mit einer Klemmfläche im Bereich zwischen den beiden Klemmflächen der Klemmbackenabschnitte angeordnet ist.

Die Klemmflächen der beiden Klemmbacken sind somit in Längsrichtung eines einzuklemmenden Brillenbügels versetzt angeordnet. Brillenbügel von modernen Brillen bestehen in der Regel aus einem elastischen Material. Durch die versetzte Anordnung der Klemmflächen wird die Elastizität der Brillenbügel genutzt, um eine feste und stabile Fixierung am Brillenbügel zu erzielen. Eine solche versetzte klemmende Anordnung der Klemmflächen hat den Vorteil, dass durch die Elastizität des Brillenbügels die Klemmung auch weiterhin Bestand hat, wenn der Klemmmechanismus bspw. etwas verrutscht oder die Backen geringfügig nachgeben. Durch die Elastizität des dazwischen eingespreizten Brillenbügels können kleine Veränderungen kompensiert werden, ohne dass sich der Klemmmechanismus löst. Sind hingegen die Klemmflächen der Klemmbacken unmittelbar einander gegenüberliegend angeordnet, dann schlagen sie an gegenüberliegenden Flächen des Brillenbügels an. Um fest daran zu sitzen, müssen die mt ihren Klemmflächen direkt einander gegenüberliegenden Klemmbacken so fest zusammengezogen werden, dass die Gefahr besteht, dass sie in den Brillenbügel einschneiden. Wenn sie nicht ausreichend fest angezogen werden und der Brillenbügel im Klemmmechanismus nur geringfügig verrutscht, dann besteht die Gefahr, insbesondere bei Brillenbügel deren Dicke sich in Längsrichtung verändert, dass sich der Klemmmechanismus löst, da der Klemmmechanismus dazu neigt in Richtung zum dünneren Bereich des Brillenbügels zu rutschen. Daher ist die versetzte Anordnung der Klemmflächen der beiden Klemmbacken sehr vorteilhaft.

Nach einem zweiten Aspekt zeichnet sich der Klemmmechanismus dadurch aus, dass die beiden Klemmbacken derart zusammensteckbar sind, dass sie in Längsrichtung der stiftförmigen Halteeinrichtung zueinander verschieblich gelagert sind.

So können zwischen den Klemmbacken unterschiedlich dicke Brillenbügel geklemmt werden, da die beiden Klemmbacken aufgrund der verschieblichen Lagerung zueinander unterschiedlich weit voneinander beabstandet werden können.

Vorzugsweise sind die beiden Klemmbacken formschlüssig zusammensteckbar ausgebildet. Durch das formschlüssige Zusammenstecken wird sichergestellt, dass Klemmflächen der Klemmbacken korrekt zueinander angeordnet sind. Der Formschluss kann ein gewisses Spiel aufweisen, sofern die Klemmflächen korrekt zueinander angeordnet sind.

Zudem ist der Klemmmechanismus sehr einfach, kompakt und dennoch stabil ausgebildet, da durch den Formschluss die Klemmbacken zueinander verdrehsicher angeordnet sind und zuverlässig einen Brillenbügel klemmen können, welcher mit unterschiedlicher Dicke und/oder unterschiedlicher Form ausgebildet sein kann. Auch wenn der Klemmmechanismus sehr kompakt ausgebildet sein kann, kann er ein hohes Drehmoment vom Schutzschild aufnehmen, so dass selbst ein großes Schutzschild, das sich um eine Lupe oder um ein Fernglas herum erstrecken kann, zuverlässig gehalten wird.

Ein erfindungsgemäßer Klemmmechanismus kann einfach an einem Brillenbügel einer Brille, insbesondere Lupenbrille, befestigt werden und gleichzeitig ein Schutzschild halten. Da die Halteeinrichtung stiftförmig ausgebildet ist, kann das Schutzschild um die stiftförmige Halteeinrichtung geschwenkt werden, so dass das Schutzschild bei Bedarf vor dem Gesicht der die Brille tragenden Person angeordnet werden kann, oder wenn kein Bedarf eines Schutzes besteht, nach oben weggeklappt werden kann. Das Schutzschild schützt den Gesichtsbereich zuverlässig vor Flüssigkeitsspritzer, insbesondere Blutspritzer, und reduziert so die Infektionsgefahr, wobei ein gleichzeitiges Tragen der Lupenbrille möglich ist.

Eine Lupenbrille im Sinne der vorliegenden Erfindung ist eine Brille mit zumindest einer Lupe oder zumindest einem Fernglas, welche vor einem Brillenglas angeordnet sind.

Die stiftförmige Halteeinrichtung ist vorzugsweise horizontal ausgerichtet. Die stiftförmige Halteeinrichtung kann ein Gewindebolzen und das Fixierelement ein Schraubenkopf oder eine Mutter sein, wobei sie derart angeordnet sind, dass durch Drehen des Schraubenkopfes oder der Mutter das Schutzschild zwischen der Klemme und dem Schraubenkopf oder der Mutter durch Klemmen fixierbar ist. Die Klemme besitzt hierdurch eine doppelte Funktion, nämlich einmal zum Klemmen des Klemmmechanismus an einen Brillenbügel und zum anderen dient es als Gegenlager zum Klemmen des Schutzschildes zwischen sich und dem Schraubenkopf bzw. der Mutter. Hierdurch ist der gesamte Klemmmechanismus sehr kompakt ausgebildet.

Der äußere Klemmbacken ist vorzugsweise größer als die innere Klemmbacken ausgebildet, so dass er eine entsprechend große Anlagefläche für das Schutzschild bildet.

Der Schraubenkopf ist vorzugsweise ein Rändelschraubenkopf oder die Mutter ist vorzugsweise eine Rändelmutter, welche einfach ohne Werkzeug betätigbar sind.

Benachbart zum Schraubenkopf oder benachbart zur Mutter kann ein Ring angeordnet sein, so dass beim Klemmen eines Schutzschildes sich der Kunststoffring zwischen dem Schraubenkopf bzw. der Mutter und dem Schutzschild befindet. Der Ring kann aus Kunststoff oder Metall ausgebildet sein. Er weist vorzugsweise eine glatte Oberfläche mit abgerundeten Kanten auf, so dass das Schutzschild nicht beschädigt wird. Hierdurch wird sichergestellt, dass das Schutzschild nicht durch den Schraubenkopf bzw. nicht durch die Mutter verkratzt wird.

Bevorzugt wird jedoch eine Schraubenkopf bzw. eine Mutter mit Bund verwendet. Ein Bund ist ein stumpfer Vorsprung im Bereich um den Gewindebolzen. Hiermit kann der Ring weggelassen werden, ohne dass das Schutzschild verkratzt wird.

Die Klemme kann einen äußeren und einen inneren Klemmbacken aufweisen, wobei der äußere Klemmbacken zur Anordnung an der Außenseite eines Brillenbügels vorgesehen ist und die stiftförmige Halteeinrichtung nach außen, insbesondere horizontal, am äußeren Klemmbacken vorsteht. "Innen" bedeutet im Sinne der vorliegenden Patentanmeldung der Bereich innerhalb eines Brillengestells, der durch das Brillengestell mit aufgeklapptem Brillenbügel begrenzt wird. "Außen" bedeutet der Bereich außerhalb des Brillengestells mit aufgeklapptem Brillenbügel.

Der äußere und der innere Klemmbacken können als separate Teile ausgebildet sein. Einer der beiden Klemmbacken, insbesondere der äußere Klemmbacken und/oder der Klemmbacken, der zwei Klemmflächen aufweist, ist vorzugsweise aus Kunststoff ausgebildet.

Ein aus Kunststoff ausgebildeter Klemmbacken besitzt eine gewisse Elastizität, so dass er sich beim Klemmen an den Brillenbügel anschmiegen kann. Hierdurch wird die Klemmwirkung verbessert.

Der äußere Klemmbacken dient als Gegenlager zum Klemmen des Schutzschildes zwischen dem Fixierelement und dem äußeren Klemmbacken. Dadurch, dass der äußere Klemmbacken aus Kunststoff ausgebildet ist, besteht einerseits ein hoher Reibschluss zwischen dem Klemmbacken und dem Schutzschild und andererseits wird ein Verkratzen des Schutzschildes vermieden.

Der äußere Klemmbacken kann jedoch auch aus Metall ausgebildet sein.

Am inneren Klemmbacken kann ein Gewinde zur Aufnahme eines als stiftförmige Halteeinrichtung dienenden Gewindebolzens ausgebildet sein. Der Gewindebolzen ist in das Gewinde des inneren Klemmbackens eingeschraubt und dient als stiftförmige Halteeinrichtung. Ein solcher innerer Klemmbacken mit Gewinde ist vorzugsweise aus Metall ausgebildet.

Am inneren Klemmbacken kann auch ein als stiftförmige Halteeinrichtung dienender Gewindebolzen angeformt sein. Der Gewindebolzen und der innere Klemmbacken kann ein einteiliges Metallteil sein. Es ist jedoch auch möglich, dass der Gewindebolzen in einen aus Kunststoff ausgebildeten inneren Klemmbacken bspw. durch Gießen formschlüssig eingesetzt ist.

Der innere und der äußere Klemmbacken sind vorzugsweise an ihren zueinander weisenden Seiten jeweils konkav geformt, so dass unterschiedlich geformte Brillenbügel sicher und fest dazwischen eingeklemmt werden können. Diese konkaven Bereiche bilden Klemmflächen.

Einer der Klemmbacken, insbesondere der äußere Klemmbacken, kann mit einer Rastnut zum Aufnehmen eines Kabels ausgebildet sein. Die Rastnut ist vorzugsweise nach oben hin offen angeordnet. Das Kabel kann zur Stromversorgung einer an der Brille angeordneten Beleuchtungseinrichtung vorgesehen sein, die mit der Rastnut am Klemmbacken fixierbar ist. Der innere Klemmbacken weist vorzugsweise einen nach außen weisenden Führungsvorsprung und der äußere Klemmbacken eine entsprechende Führungsausnehmung zum formschlüssigen Aufnehmen des Führungsvorsprungs auf. Durch das Eingreifen des Führungsvorsprungs in die Führungsausnehmung sind die beiden Klemmbacken zueinander verschieblich gelagert, so dass sie mit variablem Abstand zueinander angeordnet werden können jedoch nicht verkippen können. Im Rahmen der Erfindung ist es auch möglich, dass der äußere Klemmbacken einen nach innen weisenden Führungsvorsprung und der innere Klemmbacken eine entsprechende Führungsausnehmung zum formschlüssigen Aufnehmen des entsprechenden Führungsvorsprungs aufweist.

Vorzugsweise ist der äußere Klemmbacken in der Seitenansicht U-förmig mit zwei vertikalen Klemmbackenabschnitten ausgebildet, so dass der äußere Klemmbacken um eine an einem Brillenbügel vorgesehene Kabelklemme herum anordbar ist.

Ist ein solcher U-förmiger Klemmbacken aus einem elastischen Material, wie z.B. Kunststoff ausgebildet, dann bewirkt die U-förmige Ausgestaltung eine höhere Elastizität, wodurch die Klemmung verbessert wird und der Klemmmechanismus auch an einem sich verjüngenden Abschnitt eines Brillenbügels gut befestigt werden kann.

Brillenbügel verjüngen sich oftmals zu ihrem freien Ende hin, wobei sie Abschnitte besitzen können, an welchen sich die Querschnittsfläche stark verändert. Hier ist es schwierig eine feste Klemmung zu erzielen ohne den Brillenbügel zu beschädigen. Eine Klemmung an einem solchen sich verjüngenden Abschnitt ist durch die oben erläuterte versetzte Anordnung der Klemmflächen möglich, mit welchen eine 3-Punkt-Klemmung erzielt wird. Die U-förmige Ausgestaltung des Klemmbackens und/oder die elastische Ausbildung des zwei Klemmflächen aufweisenden Klemmbackens verbessert die Klemmwirkung weiter.

Nach einem weiteren Aspekt der Erfindung ist eine Schutzschildvorrichtung zur Befestigung eines Schutzschildes an einer Brille vorgesehen. Die Brille ist insbesondere eine Lupenbrille. Die Schutzschildvorrichtung umfasst ein Schutzschild aus einem durchsichtigen Kunststoffmaterial und zwei Klemmmechanismen zum Befestigen an jeweils einem von zwei Bügeln der Brille, wobei die Klemmmechanismen den oben erläuterten Ausführungen entsprechen.

Das Schutzschild kann bspw. aus PET (Polyethylenterephthalat), PETG (mit Glykol modifiziertes PET), PS (Polystyrol), PC (Polycarbonat), PMMA (Polymethymetacrylat), SAN (Styrol Acrynitril-Copolymer), COP (Cycloolefin-Polymer), COC (Cycloolefin-Copolymer), PMMI (Polymethacrylmethylimid) oder FMS (Polytrifluormethylstyrol) ausgebildet sein.

Das Schutzschild ist vorzugsweise aus einer flexiblen Kunststofffolie ausgebildet, so dass es an unterschiedlich große Brillen einfach angepasst werden kann. Das Schutzschild weist vorzugsweise einen Frontbereich und zwei Seitenlaschen auf, die sich vom Frontbereich nach hinten etwa parallel zu den Brillenbügeln erstrecken. Die Seitenlaschen können mit jeweils einem oder mehreren Durchgangslöchern versehen sein, welche von der stiftförmigen Halteeinrichtung durchgriffen werden. Das Vorsehen mehrerer Löcher an den Seitenbereichen dient zur unterschiedlichen Positionierung des Schutzschildes bezüglich der Brille und erlaubt eine individuelle Anpassung der Positionierung des Schutzschildes an die Bedürfnisse des Benutzers.

Nach einem weiteren Aspekt der vorliegenden Erfindung wird eine Brille, insbesondere eine Lupenbrille, mit einer oben erläuterten Schutzschildvorrichtung vorgesehen. Die Klemmmechanismen sind jeweils an einem von zwei Bügeln der Brille befestigt, um das Schutzschild schwenkbar und feststellbar zu halten.

Die Brille ist vorzugsweise eine Lupenbrille mit zwei Brillengläsern, wobei an oder vor zumindest einem der Brillengläser eine Lupe oder ein Fernrohr angeordnet ist.

Die Brille kann auch mit einer Beleuchtungseinrichtung versehen sein.

Nachfolgend wird die Erfindung beispielhaft näher anhand der Zeichnungen erläutert. Die Zeichnungen zeigen beispielhaft in:
- Figur 1: eine Lupenbrille mit einem Schutzschild im Gebrauch in der Seitenansicht,
- Figur 2: einen Teil der Brille aus Figur 1 mit einem Klemmmechanismus und einem Schutzschild in perspektivischer Ansicht,
- Figur 3: die Brille aus Figur 1 mit Schutzschild im Gebrauch, wobei das Schutzschild hochgeklappt ist, in einer Seitenansicht,
- Figur 4a und 4b: den in den Figuren 1 - 3 gezeigten Klemmmechanismus in einer Explosionsdarstellung und im zusammengesetzten Zustand in perspektivischer Ansicht,
- Figur 5: eine weitere Ausführungsform des Klemmmechanismus ohne Rastnut zur Aufnahme eines Kabels, und
- Figur 6: eine weitere Ausführungsform eines Klemmmechanismus in teilweiser Explosionsansicht.

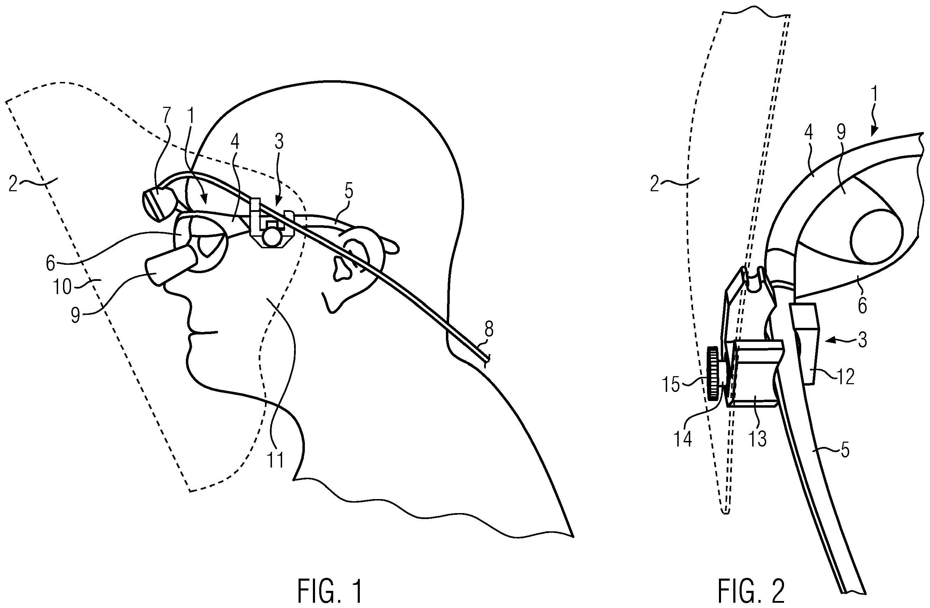

Zur Reduktion der Ansteckungsgefahr bei Operationen und Diagnosen am Patienten wird eine Brille 1 mit einem Schutzschild 2 vorgesehen, das mittels zweier Klemmmechanismen 3 1 an der Brille 1 befestigt ist (Figur 1-3).

Eine Brille besteht grundsätzlich aus einem Brillengestell 4 mit zwei Brillenbügeln 5 und zwei Brillengläsern 6. Die Brillenbügel 5 sind in üblicher Weise schwenkbar an der Brille ausgebildet. Die Brille 1 ist eine Lupenbrille, d.h., dass an oder vor den Brillengläsern jeweils eine Lupe oder ein Fernrohr angeordnet ist, durch die der Benutzer der Brille beim Tragen der Brille hindurchsehen kann. Optional kann eine Beleuchtungseinrichtung 7 an der Brille 1 angeordnet sein, welche mittels eines Kabels 8 mit Strom versorgt werden kann. Das Kabel 8 kann jedoch auch dazu vorgesehen sein, um Bilddaten, welche mit einer in der Lupe oder im Fernrohr 9 integrierten Kamera erfasst werden.

Das Schutzschild 2 ist aus einer Kunststofffolie mit einer Dicke von zumindest etwa 0,3 mm, insbesondere zumindest 0,5 mm bzw. zumindest 0,60 mm ausgebildet. Hierdurch hat das Schutzschild eine ausreichende Festigkeit. Die maximale Dicke beträgt 4 mm bzw. 3 mm und insbesondere 2,5 mm. Hierdurch wird eine ausreichende Biegsamkeit des Schutzschildes sichergestellt. Die Dicke des Schutzschildes hängt auch von der Festigkeit bzw. Biegsamkeit des verwendeten Kunststoffmaterials ab. Der Kunststoff ist durchsichtig.

Das Schutzschild hat einen Frontbereich 10, an den sich seitlich zwei Seitenlaschen 11 anschließen. In den Seitenlaschen 11 sind mehrere Durchgangslöcher ausgebildet, um das Schutzschild 2 an den Klemmmechanismen 3 befestigen zu können. Das Schutzschild 2 bildet somit eine durchsichtige Schutzscheibe.

Im vorliegenden Ausführungsbeispiel ist das Schutzschild aus PETG, d.h., aus einem mit Glykol modifizierten Polyethylentherephthalat, ausgebildet, das sehr transparent und extrem feinporig ist, so dass es eine sehr glatte Oberfläche besitzt, welche einfach und zuverlässig auch von Schmutz, Viren und Keimen gereinigt und desinfiziert werden kann.

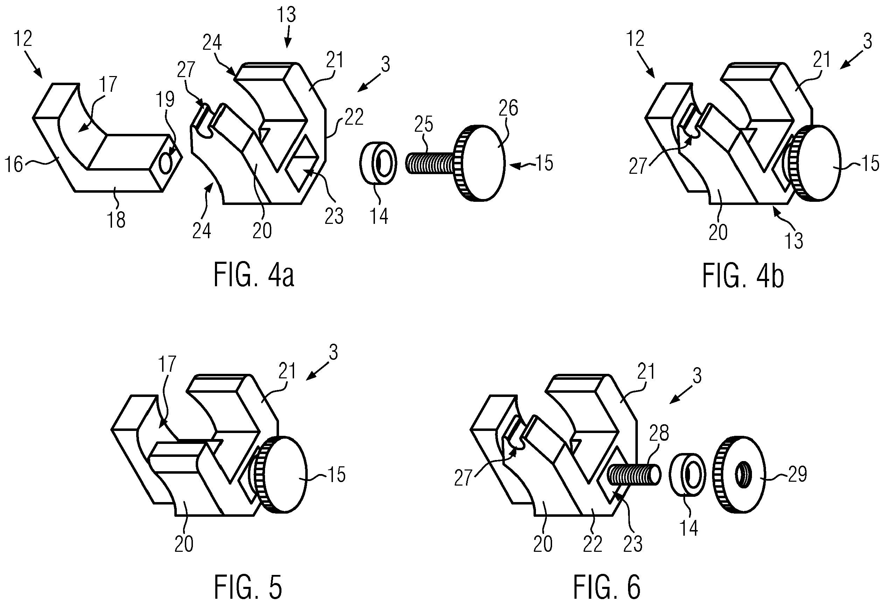

Der Klemmmechanismus 3 (Figur 4a, 4b) der Brille 1 (Figur 1 - 3) umfasst einen inneren Klemmbacken 12, einen äußeren Klemmbacken 13, einen Kunststoffring 14 und eine Rändelschraube 15.

Der innere Klemmbacken 12 weist einen Klemmbackenabschnitt 16 mit einer konkaven Klemmfläche 17 und einem etwa rechtwinklig an einem Rand des Klemmbackenabschnittes 16 angeformten Führungsvorsprung 18 auf. Der Führungsvorsprung 18 besitzt einen etwa rechteckförmigen Querschnitt und weist eine an der Stirnseite des Führungsvorsprungs 18 mündende Bohrung 19 auf, in welcher ein Innengewinde ausgebildet ist. Der innere Klemmbacken 12 ist aus Metall ausgebildet.

Der äußere Klemmbacken 13 ist in der Seitenansicht U-förmig ausgebildet mit einem vorderen und einem hinteren Klemmbackenabschnitt 20, 21 und einem Basisabschnitt 22, der die beiden Klemmbackenabschnitte 20, 21 an dem unteren Rand miteinander verbindet. Der Basisabschnitt 22 besitzt eine im Querschnitt rechteckförmige Führungsausnehmung, welche im vorliegenden Ausführungsbeispiel (Figur 4a) ein Durchgangsloch ist. Die Führungsausnehmung 23 weist einen rechteckförmigen Querschnitt auf, der komplementär zum rechteckförmigen Querschnitt des Führungsvorsprungs 18 des inneren Klemmbackens 12 geformt ist, so dass der Führungsvorsprung 18 mit geringem Spiel in der Führungsausnehmung 23 verschieblich gelagert ist.

Die beiden Klemmbackenabschnitte 20, 21 des äußeren Klemmbacken 13 besitzen konkave Klemmflächen 24, welche zum inneren Klemmbacken 12 weisend angeordnet sind.

Die Rändelschraube weist einen Gewindebolzen 25 mit einem Gewinde, das in kämmenden Eingriff mit dem Innengewinde der Bohrung 19 des inneren Klemmbackens 12 treten kann. Die Rändelschraube 15 besitzt einen Schraubenkopf 26, welcher am Umfang geriffelt ist, so dass er ohne Werkzeug betätigt werden kann.

Wie es in Figur 2 gezeigt ist, wird ein Brillenbügel 5 zwischen den Klemmflächen des inneren Klemmbackens 12 und des äußeren Klemmbackens 13 angeordnet, die Klemmschraube 15 durch ein Loch im Schutzschild 2 gesteckt und in das Gewinde der Bohrung 19 des inneren Klemmbackens 12 eingeschraubt, wobei der Kunststoffring 14 zwischen dem Schraubenkopf 26 und dem Schutzschild 2 angeordnet wird. Durch Eindrehen der Rändelschraube 15 in das Gewinde des inneren Klemmbackens 12 werden einerseits der innere Klemmbacken 12 und der äußere Klemmbacken 13 zusammengedrückt, wodurch der Klemmmechanismus 3 am Brillenbügel 4 fixiert wird und andererseits wird gleichzeitig das Schutzschild 2 zwischen dem Kunststoffring 14 und dem äußeren, aus Kunststoff ausgebildeten Klemmbacken 13 eingeklemmt und in ihrer Position fixiert.

Mit diesem einfachen Klemmmechanismus 3 kann dieser somit sowohl am Brillenbügel 5 als auch gleichzeitig das Schutzschild 2 am Klemmmechanismus 3 fixiert werden.

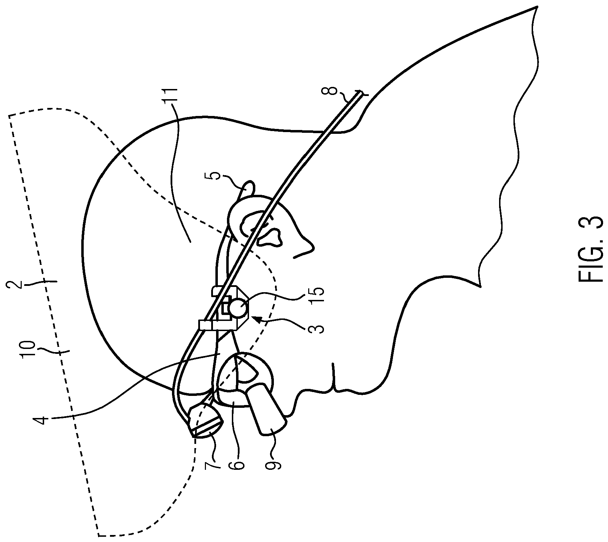

Löst man den Klemmmechanismus 3 etwas durch Herausdrehen der Rändelschraube 15, dann kann man das Schutzschild 2 am Klemmmechanismus 3 schwenken. In Figur 1 ist das Schutzschild 2 in einer Schutzposition dargestellt, in welcher sie das gesamte Gesicht des Benutzers abdeckt, wobei zwischen dem Schutzschild 2 und der Brille 1 ausreichend Platz ist, dass auch die Lupe bzw. das Fernrohr 9 mit einer Länge von einigen Zentimetern Platz haben. Das Schutzschild 2 kann nach oben weggeschwenkt werden, wie es in Figur 3 gezeigt ist. Das Schutzschild 2 kann in jeder beliebigen Schwenkposition durch Eindrehen der Rändelschraube 15 fixiert werden.

Ein Arzt, der die Brille 1 trägt, kann somit bei Bedarf das Schutzschild 2 vor sein Gesicht schwenken und in dieser Schutzposition fixieren und das Schutzschild nach oben wegschwenken, wenn er sich beispielsweise vom Patienten wegwendet und kein Bedarf an einem Schutz besteht. Er braucht hierzu die Brille nicht abzunehmen.

Es gibt Lupenbrillen mit Kabelklemmen, welche seitlich nach außen an einem Brillenbügel 5 abstehen. Die U-förmige Ausgestaltung des äußeren Klemmbackens 13 erlaubt es, den äußeren Klemmbacken 13 um eine solche Kabelklemme herum anzuordnen, wobei der vordere Klemmbackenabschnitt 20 vor und der hintere Klemmbackenabschnitt 21 hinter der Kabelklemme positioniert wird. Zudem bewirkt das Vorsehen zweier Klemmbackenabschnitte 20, 21, welche zueinander etwas beabstandet sind, dass der Klemmmechanismus 3 beim Festklemmen am Brillenbügel 5 nicht um eine vertikale Achse verkippt. Der Klemmmechanismus 3 wird somit sehr stabil am Brillenbügel 5 fixiert.

Der Klemmmechanismus 3 gemäß dem vorliegenden Ausführungsbeispiel weist eine nach oben hin offene Rastnut 27 auf, welche zur Fixierung des Kabels 8 am Klemmmechanismus 3 dient (Figur 1 und 4a, 4b).

Ein solcher Klemmmechanismus kann auch ohne Rastnut ausgebildet sein (Figur 5). Üblicherweise ist es zweckmäßig an einer Brille 1 einen Rastmechanismus mit Rastnut zur Aufnahme eines Kabels und einen anderen Klemmmechanismus ohne einer solchen Rastnut vorzusehen, da in der Regel nur ein einziges Kabel benötigt wird. Grundsätzlich ist es jedoch auch möglich, auf beiden Seiten der Brille einen Klemmmechanismus 3 mit jeweils einer Rastnut 27 vorzusehen, so dass der Benutzer der Brille frei wählen kann, ob er das Kabel am linken oder am rechten Brillenbügel 5 fixiert.

Gemäß einer weiteren Ausführungsform des Klemmmechanismus 3 kann anstelle einer Rändelschraube ein Gewindebolzen 28 und eine separat ausgebildete Mutter 29, insbesondere eine Rändelmutter 29, vorgesehen sein (Figur 6). Der Gewindebolzen 28 ist einteilig am inneren Klemmbacken 12 angeformt. Der innere Klemmbacken 12 kann ein Kunststoffteil sein, in dem der Gewindebolzen 28 eingegossen ist. Der Gewindebolzen 28 und der innere Klemmbacken 12 kann auch ein einstückiges Metallteil sein. Eine solche Ausführungsform des Klemmmechanismus 3 ist in der Fertigung aufwändiger als die in den Figuren 4a - 5 gezeigten Klemmmechanismen 3, hat jedoch den Vorteil, dass der Gewindebolzen 28 im noch nicht endgültig verschraubten Zustand am Klemmmechanismus 3 horizontal nach außen vorsteht, so dass das Schutzschild 2 einfach auf den Gewindebolzen 28 gesteckt werden kann. Bei den in Figur 4a - 5 gezeigten Ausführungsformen muss die Rändelschraube durch die entsprechende Durchgangsöffnung in das Schutzschild 2 zunächst durchgeführt und dann in das Gewinde des inneren Klemmbackens 12 eingeschraubt werden.

Die oben gezeigten Ausführungsformen der Klemmmechanismen sind sehr einfach ausgebildet und können kostengünstig hergestellt werden. Dadurch, dass das Schutzschild 2 zwischen zwei Kunststoffteilen (äußere Klemmbacken 13, Kunststoffring 14) eingeklemmt wird, wird ein Verkratzen des Schutzschildes 2 vermieden. Anstelle von Kunststoffteilen können auch Metallkörper mit stumpfer glatter Oberfläche, wie z.B. eine Rändelmutter mit Bund, verwendet werden, um die Gefahr eines Verkratzens des Schutzschildes 2 zu vermeiden. Das Schutzschild 2 kann in beliebigen Schwenkstellungen angeordnet und fixiert werden. Der Klemmmechanismus 3 ist sehr klein und leicht. Ein Benutzer kann das Schutzschild 2 vor sein Gesicht schwenken, so dass es vollständig geschützt ist. Der Abstand zwischen das Schutzschild 2 und dem Gesicht ist so groß, dass der Benutzer frei atmen kann, ohne dass die Gefahr besteht, dass das Schutzschild beschlägt. Das Schutzschild kann, wenn kein Bedarf nach einem Schutz besteht, nach oben weggeschwenkt werden, ohne dass die Brille abgenommen werden muss. Das Schutzschild 2 beeinträchtigt in keinster Weise die Funktion der Lupe bzw. des Fernrohres, der Beleuchtungseinrichtung oder einer Kamera.

Das Schutzschild kann kontinuierlich verwendet werden und ist einfach und zuverlässig zu reinigen.

Beim oben erläuterten Ausführungsbeispiel ist der Klemmmechanismus aus zwei Klemmbacken 12, 13 ausgebildet, welche jeweils separate Teile sind. Im Rahmen der Erfindung ist es auch möglich, dass die beiden Klemmbacken 12, 13 Bestandteil eines insbesondere elastischen Kunststoffteils sind, das alleine aufgrund der Federwirkung des Materials an einen Brillenbügel geklemmt werden kann. Bei einer solchen Ausgestaltung des Klemmmechanismus ist es zweckmäßig, wenn der Verbindungsabschnitt zwischen dem inneren Klemmbacken und dem äußeren Klemmbacken oberhalb des Brillenbügels angeordnet wird, so dass sichergestellt ist, dass der Klemmmechanismus nicht nach unten abrutschen kann.

Beim oben beschriebenen Ausführungsbeispiel ist am inneren Klemmbacken ein Gewinde ausgebildet. Die beiden Klemmbacken können grundsätzlich auch ohne Gewinde ausgebildet sein, wobei zum Fixieren derselben dann bspw. eine Schraube mit einem Schraubenkopf und eine Mutter verwendet wird, wobei sich der Gewindebolzen der Schraube durch beide Klemmbacken hindurch erstreckt.

**Bezugszeichenliste**

| | |
|---|---|
| 1 Brille | 16 Klemmbackenabschnitt |
| 2 Schutzschild | 17 Klemmfläche |
| 3 Klemmmechanismus | 18 Führungsvorsprung |
| 4 Brillengestell | 19 Bohrung |
| 5 Brillenbügel | 20 Vorderer Klemmbackenabschnitt |
| 6 Brillenglas | 21 Hinterer Klemmbackenabschnitt |
| 7 Beleuchtungseinrichtung | 22 Basisabschnitt |
| 8 Kabel | 23 Führungsausnehmung |
| 9 Lupe/Fernerohr | 24 Klemmfläche |
| 10 Frontbereich | 25 Gewindebolzen |
| 11 Seitenlasche | 26 Schraubenkopf |
| 12 Innerer Klemmbacken | 27 Rastnut |
| 13 Äußerer Klemmbacken | 28 Gewindebolzen |
| 14 Kunststoffring | 29 Mutter |
| 15 Rändelschraube | |

## Patentansprüche

1. Klemmmechanismus zum Befestigen eines Schutzschildes (2) an einer Brille (3), insbesondere nach Anspruch 1, umfassend
eine Klemme (12, 13) zum Klemmen an einen Brillenbügel (5), wobei die Klemme einen inneren und einen äußeren Klemmbacken (12, 13) aufweist und der äußere Klemmbacken (13) zur Anordnung an der Außenseite eine Brillenbügels (5) vorgesehen ist,
eine stiftförmige Halteeinrichtung (25, 28) zum Durchgreifen einer Durchgangsbohrung in einem Schutzschild (2), und
ein Fixierelement (26, 29) zum Fixieren des Schutzschildes (2) am Klemmmechanismus (3),
**dadurch gekennzeichnet,**
**dass** einer der beiden Klemmbacken (12, 13) und insbesondere der äußere Klemmbacken (13) mit zwei vertikalen Klemmbackenabschnitten (20, 21) ausgebildet ist, welche jeweils eine Klemmfläche (24) bilden, so dass der andere Klemmbacken (13, 12) mit einer Klemmfläche (17) im Bereich zwischen den beiden Klemmflächen (24) der Klemmbackenabschnitte (20, 21) angeordnet ist.

2. Klemmmechanismus zum Befestigen eines Schutzschildes (2) an einer Brille (3), umfassend
eine Klemme (12, 13) zum Klemmen an einen Brillenbügel (5), wobei die Klemme einen inneren und einen äußeren Klemmbacken (12, 13) aufweist und der äußere Klemmbacken (13) zur Anordnung an der Außenseite eine Brillenbügels (5) vorgesehen ist,
eine stiftförmige Halteeinrichtung (25, 28) zum Durchgreifen einer Durchgangsbohrung in einem Schutzschild (2), und
ein Fixierelement (26, 29) zum Fixieren des Schutzschildes (2) am Klemmmechanismus (3),
**dadurch gekennzeichnet,**
**dass** die beiden Klemmbacken (12, 13) derart zusammensteckbar sind, dass sie in Längsrichtung der stiftförmigen Halteeinrichtung (25, 28) zueinander verschieblich gelagert sind.

3. Klemmmechanismus nach Anspruch 1 oder 2,
**dadurch gekennzeichnet,**
**dass** die stiftförmige Halteeinrichtung ein Gewindebolzen (25, 28) und das Fixierelement ein Schraubenkopf (26) oder eine Mutter (29) ist, wobei sie derart angeordnet sind, dass durch Drehen des Schraubenkopfes (26) oder der Mutter (29) das Schutzschild (2) zwischen der Klemme (12, 13) und dem Schraubenkopf (26) oder der Mutter (29) durch Klemmen fixierbar ist.

4. Klemmmechanismus nach Anspruch 3,
**dadurch gekennzeichnet,**
**dass** der Schraubenkopf (26) ein Rändelschraubenkopf oder die Mutter (29) eine Rändelmutter ist.

5. Klemmmechanismus nach Anspruch 3 oder 4,
**dadurch gekennzeichnet,**
**dass** benachbart zum Schraubenkopf (26) oder benachbart zur Mutter (29) ein Ring (14) angeordnet ist, so dass beim Klemmen eines Schutzschildes (2) sich der Ring (14) zwischen dem Schraubenkopf (26) bzw. der Mutter (29) und dem Schutzschild (2) befindet, und/oder dass die stiftförmige Halteeinrichtung (25, 28) nach außen am äußeren Klemmbacken (13) vorsteht, und/oder dass der äußere Klemmbacken (13) und/oder der Ring (14) aus Kunststoff ausgebildet sind.

6. Klemmmechanismus nach einem der Ansprüche 1 bis 5,
**dadurch gekennzeichnet,**
**dass** der innere Klemmbacken (12) entweder ein Gewinde zur Aufnahme eines als stiftförmige Halteeinrichtung dienenden Gewindebolzen (25, 28) aufweist oder ein als stiftförmige Halteeinrichtung dienender Gewindebolzen (28) daran angeformt ist.

7. Klemmmechanismus nach einem der Ansprüche 1 bis 6,
**dadurch gekennzeichnet,**
**dass** der innere Klemmbacken (12) aus Metall oder Kunststoff ausgebildet ist.

8. Klemmmechanismus nach einem der Ansprüche 1 bis 7,
**dadurch gekennzeichnet**,
die Klemmbacken (12, 13) an ihren zueinander weisenden Seiten jeweils konkav geformte Klemmflächen (17, 24) aufweisen, und/oder dass an einem der Klemmbacken (13) eine Rastnut (27) zum Aufnehmen eines Kabels (8) ausgebildet ist.

9. Klemmmechanismus nach einem der Ansprüche 1 bis 8,
**dadurch gekennzeichnet,**
**dass** einer der beiden Klemmbacken (12) einen zum anderen Klemmbacken (13) weisenden Führungsvorsprung (18) und der andere Klemmbacken (13) eine entsprechende Führungsausnehmung (23) zum formschlüssigen Aufnehmen des Führungsvorsprungs (18) aufweist.

10. Klemmmechanismus nach einem der Ansprüche 1 bis 9,
**dadurch gekennzeichnet,**
**dass** einer der beiden Klemmbacken (12, 13) und insbesondere der äußere Klemmbacken (13) in der Seitenansicht U-förmig mit zwei vertikalen Klemmbackenabschnitten (20, 21) ausgebildet ist.

11. Schutzschildvorrichtung zur Befestigung eines Schutzschildes (2) an einer Brille (3), insbesondere an einer Lupenbrille, umfassend
ein Schutzschild (2) aus einem durchsichtigem Kunststoffmaterial und zwei Klemmmechanismen (3) zum Befestigen an jeweils einem von zwei Bügeln (5) der Brille (1), wobei die Klemmmechanismen (3) nach einem der Ansprüche 1 bis 10 ausgebildet sind.

12. Schutzschildvorrichtung nach Anspruch 11,
**dadurch gekennzeichnet,**
**dass** das Schutzschild (2) aus PET (Polyethylenterephthalat), PETG (mit Glykol modifiziertes PET), PS (Polystyrol), PC (Polycarbonat), PMMA (Polymethymetacrylat), SAN (Styrol Acrynitril-Copolymer), COP (Cycloolefin-Polymer), COC (Cycloolefin-Copolymer), PMMI (Polymethacrylmethylimid) oder FMS (Polytrifluormethylstyrol) ausgebildet ist.

13. Schutzschildvorrichtung nach Anspruch 11 oder 12,
**dadurch gekennzeichnet,**
**dass** das Schutzschild (2) aus einer flexiblen Kunststofffolie ausgebildet ist, und/oder dass das Schutzschild (2) mehrere Durchgangslöcher aufweist, um jeweils von der stiftförmigen Halteeinrichtung (25, 28) durchgriffen zu werden.

14. Lupenbrille mit zwei Brillengläser (6), wobei an oder vor zumindest einem der Brillengläser (6) eine Lupe oder ein Fernrohr (9) angeordnet ist und mit einer Schutzschildvorrichtung umfassend ein Schutzschild (2) aus einem durchsichtigem Kunststoffmaterial und zwei Klemmmechanismen zum Befestigen des Schutzschildes (2) an einer Brille (3), umfassend
eine Klemme (12, 13) zum Klemmen an einen Brillenbügel (5), eine stiftförmige Halteeinrichtung (25, 28) zum Durchgreifen einer Durchgangsbohrung in einem Schutzschild (2), und
ein Fixierelement (26, 29) zum Fixieren des Schutzschildes (2) am Klemmmechanismus (3), wobei
jeweils einer der Klemmmechanismen (3) an einem von zwei Bügeln (5) der Brille (1) befestigt ist, um das Schutzschild (2) schwenkbar und feststellbar zu halten.

15. Lupenbrille nach Anspruch 13,
**dadurch gekennzeichnet,**
**dass** an der Brille (1) eine Beleuchtungseinrichtung (7) angeordnet ist, und/oder dass die Schutzschildvorrichtung nach einem der Ansprüche 10 bis 12 ausgebildet ist.
